# EUROPÄISCHE PATENTANMELDUNG

(11) **EP 2 818 167 A1**
(43) Veröffentlichungstag der Anmeldung: **31.12.2014**
(21) Anmeldenummer: 13174235.5
(22) Anmeldetag: 28.06.2013
(51) Int. Cl.: A61K 31/439, A61K 31/46, A61K 33/28, A61P 31/12, A61P 31/16, A61P 31/22

(54) **Antiviral wirksame pharmazeutische Zusammensetzung**

(71) Anmelder: Medice Arzneimittel Pütter GmbH & Co. KG, 58638 Iserlohn (DE)
(72) Erfinder: Pütter, Sigurd, Dr., 58638 Iserlohn (DE); Ammer, Richard, Dr., Dr., 58644 Iserlohn (DE); Schmidbauer, Michael, Dr., 42283 Wuppertal (DE)
(74) Vertreter: Isarpatent

(57) **Zusammenfassung**

Die vorliegende Erfindung betrifft eine pharmazeutische Zusammensetzung umfassend Atropin oder ein Salz hiervon; Aconitin und/oder Quecksilbercyanid zur Verwendung in einem Verfahren zur Behandlung einer Virusinfektion.

## Beschreibung

### Gebiet der Erfindung

Die vorliegende Erfindung betrifft eine pharmazeutische Zusammensetzung umfassend Atropin oder ein Salz hiervon; Aconitin und/oder Quecksilbercyanid zur Verwendung in einem Verfahren zur Behandlung einer Virusinfektion.

### Hintergrund der Erfindung

Viren sind die Haupterreger von Erkältungen. Unter ihnen nehmen Rhinoviren und Coronaviren, die für 50-70 % aller Atemwegsinfekte verantwortlich sind, die vorderen Plätze ein, gefolgt von Parainfluenzaviren, RSV, Adeno- und Enteroviren. Die Influenza, eine weitere akute Atemwegserkrankung, wird ebenfalls von Viren verursacht. Obendrein profitieren Herpes-simplex-Viren (HSV) von der einhergehenden Abwehrschwäche und können unter Umständen entsprechende Symptome wie Herpes labialis hervorrufen.

Während zur Behandlung von bakteriellen Infektionen eine Fülle von Antibiotika existieren, steht zur Behandlung virusbedingter Erkrankungen nur eine relativ kleine Anzahl von Virostatika gegen ein begrenztes Virenspektrum zur Verfügung. Die mit vielen Nebenwirkungen behafteten Antibiotika tragen verständlicherweise noch weniger zur Heilung der meist viral bedingten Erkältung bei, wirken sie doch lediglich gegen die möglichen, mit einer langwierigen Erkältung einhergehenden bakteriellen Sekundärinfektionen. Die oft übereilte Verordnung von Antibiotika im großen Stil ist zudem mit einer hohen Rate an Resistenzbildung assoziiert bzw. wirkt in einer großen Anzahl von Fällen überhaupt nicht. Wünschenswert sind somit effektive und verträgliche Alternativen.

Phytotherapeutika und Komplexhomöopathika können eine solche Alternative darstellen. In der Tat wurden und werden solche Alternativen immer wieder diskutiert, da sie gegenüber den synthetischen Heilmitteln Vorteile in Gestalt einer größeren therapeutischen Breite, einer besseren Verträglichkeit und weniger Arzneimittelwechselwirkungen besitzen sowie nicht der Gefahr der Resistenzbildung ausgesetzt sind.

In den letzten Jahren sind vermehrt Studien publiziert worden, in denen eine antivirale Wirkung von pflanzenbasierten Arzneimitteln nachgewiesen werden konnte. Das Komplexhomöopathikum Meditonsin^{®} besteht aus den pflanzlichen Komponenten Atropin und Aconitin sowie dem Salz Quecksilbercyanid (Mercurius cyanatus) und wird bereits seit über 60 Jahren zur Behandlung von Entzündungen des Hals, Nasen- und Rachenraums verwendet. Klinische Untersuchungen bestätigen die Wirksamkeit bei der Behandlung von Entzündungen der oberen Atemwege und die gute Verträglichkeit.

Die der vorliegenden Erfindung zugrunde liegenden Studien haben überraschenderweise ergeben, dass Atropin, Aconitin und Quecksilbercyanid sowie daraus hergestellte Mischungen, antivirale Eigenschaften aufweisen. Besonderes Augenmerk wurde auf die Frage gelegt, ob, in welchen Konzentrationen und gegen welche Viren die einzelnen Komponenten wirken und welche Komponenten darin für die antivirale Wirkung der Mischung verantwortlich sind. Getestet wurde dabei gegen Erkältungs-, Influenza- und Herpesviren.

Die vorliegende Patentanmeldung betrifft somit die überraschende Erkenntnis, dass eine pharmazeutische Zusammensetzung die Atropin oder ein Salz hiervon, Aconitin und/oder Quecksilbercyanid umfasst in vorteilhafter Weiser zur Behandlung von Virusinfektionen eingesetzt werden kann.

### Zusammenfassung der Erfindung

Die vorliegende Erfindung zeigt die antiviralen Eigenschaften der Wirkstoffe Atropin, Aconitin und Quecksilbercyanid in verschiedenen Konzentrationsbereichen. Dabei konnte nachgewiesen werden, dass sowohl das Gemisch als auch die Einzelkomponenten die Aktivität aller getesteten Erkältungs-, Influenza- und Herpesviren wenigstens schwach bis sehr stark hemmten.

Die vorliegende Erfindung betrifft somit eine pharmazeutische Zusammensetzung umfassend Atropin oder ein Salz hiervon; Aconitin und/oder Quecksilbercyanid zur Verwendung in einem Verfahren zur Behandlung einer Virusinfektion.

### Kurze Beschreibung der Figuren

In den Figuren sind die Ergebnisse der antiviralen In-vitro-Prüfung der Prüfsubstanzen Atropinsulfat, Aconitin und Quecksilbercyanid sowie einer Mischung aus den Prüfsubstanzen abhängig von der eingesetzten Konzentration dargestellt.
Fig. 1 zeigt die relative Hemmung verschiedener Viren durch eine Atropinsulfatlösung in abnehmender Konzentration des Wirkstoffes;
Fig. 2 zeigt die relative Hemmung verschiedener Viren durch eine Aconitinlösung in abnehmender Konzentration des Wirkstoffes;
Fig. 3 zeigt die relative Hemmung verschiedener Viren durch eine Quecksilbercyanidlösung in abnehmender Konzentration des Wirkstoffes; und
Fig. 4 zeigt die relative Hemmung verschiedener Viren durch ein Gemisch von Atropinsulfat, Aconitin und Quecksilbercyanid. Die Ausgangsmischung enthielt die Prüfsubstanzen in folgenden Konzentrationen: Atropinsulfat 5 µg/ml, Aconitin µg/ml, Quecksilbercyanid 0,004 µg/ml und wurde dann auf die angegebenen Gesamtkonzentrationen verdünnt.

### Ausführliche Beschreibung der Erfindung

Die vorliegende Erfindung betrifft eine pharmazeutische Zusammensetzung umfassend oder bestehend aus a) Atropin oder einem Salz hiervon; b) Aconitin; und/oder c) Quecksilbercyanid zur Verwendung in einem Verfahren zur Behandlung einer Virusinfektion.

Der Begriff "pharmazeutische Zusammensetzung" wie hierin verwendet, umfasst insbesondere perorale Darreichungsformen, beispielsweise feste, halbflüssige oder flüssige Zusammensetzungen zur oralen Verabreichung. Eine wässrig/ethanolische Lösung zur tropfenweisen Verabreichung hat sich hierbei als besonders vorteilhaft herausgestellt. Beispielsweise werden Gemische von Ethanol 94 % (g/g), Glycerol 85 % sowie gereinigtem Wasser im Gewichtsverhältnis 5:10:85 besonders vorteilhaft eingesetzt.

Alternativ sind jedoch auch andere Darreichungsformen möglich, beispielsweise feste perorale Darreichungsformen wie Pulver, Tabletten oder Kapseln. Die Herstellung erfolgt insbesondere nach den Regeln für die Herstellung homöopathischer Arzneimittel gemäß Homöopathischem Arzneibuch 2012 (HAB 2012), amtliche Ausgabe. Beispielsweise können Verreibungen nach Vorschrift des HAB hergestellt werden, bei denen die Ausgangsstoffe stufenweise mit Lactose-Monohydrat verrieben werden. Alternativ können Tabletten aus den entsprechenden Verreibungen verpresst werden. Hierbei können begrenzte Zusätze von Magnesiumstearat oder Calciumbehenat als Gleitmittel und von Stärke als Zerfallsbeschleuniger verwendet werden. Im Falle der Granulation können wässrige Lactose-Lösung, Ethanol und geeignete Konzentrationen von Stärkekleistern eingesetzt werden.

Zudem können Streukügelchen nach der HAB-Vorschrift 10 hergestellt werden, wobei Zuckerkügelchen mit 1/100 Massenteilen der betreffenden Dilution gleichmäßig befeuchtet und an der Luft getrocknet werden.

Neben peroralen Arzneiformen kann die pharmazeutische Zusammensetzung der vorliegenden Erfindung Parenteralia, d.h. flüssige Verdünnungen zur Injektion sowie flüssige Einreibungen sowie Salben, Suppositorien, Augentropfen, Nasentropfen umfassen, die nach den HAB-Vorschriften 11 - 15 sowie 45 hergestellt wurden. Wie oben ausgeführt, handelt es sich bei der vorliegenden pharmazeutischen Zusammensetzung in der am meisten bevorzugten Ausführungsform um eine perorale Lösung, die nach den Vorschriften des Homöopathischen Arzneibuchs hergestellt wurde.

Einer der möglichen Bestandteile der pharmazeutischen Zusammensetzung ist Atropin. Atropin ist ein Tropan-Alkaloid, bei dem es sich um ein Racemat aus den Isomeren (R)- und (S)-Hyoscyamin entsprechend der folgenden Strukturformel handelt. Die (R)-Form ist oben, die (S)-Form unten angegeben:

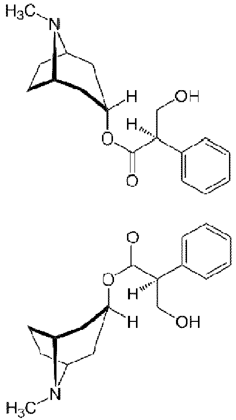

Atropin gehört zu den Parasympatholytika und konkurriert an den muskarinischen Rezeptoren des Parasympathikus mit dem Neurotransmitter Acetylcholin. Die körperlichen Wirkungen der Einnahme von Atropin sind wie folgt: Beschleunigung der Herzfrequenz, Weitstellung der Bronchien, verminderte Speichelbildung u.v.a. Darüber hinaus wurden weitere, jedoch seltene Anwendungsgebiete beschrieben, beispielsweise die Anwendung bei Krämpfen des Magen-Darmtraktes, bei erschwerter Blasenentleerung sowie Harninkontinenz. Eine Verwendung als Antivirusmittel ist jedoch bislang nicht beschrieben worden.

Bei dem erfindungsgemäß eingesetzten Salz des Atropins handelt es sich üblicherweise um Atropinsulfat, beispielsweise Atropinsulfat-Monohydrat.

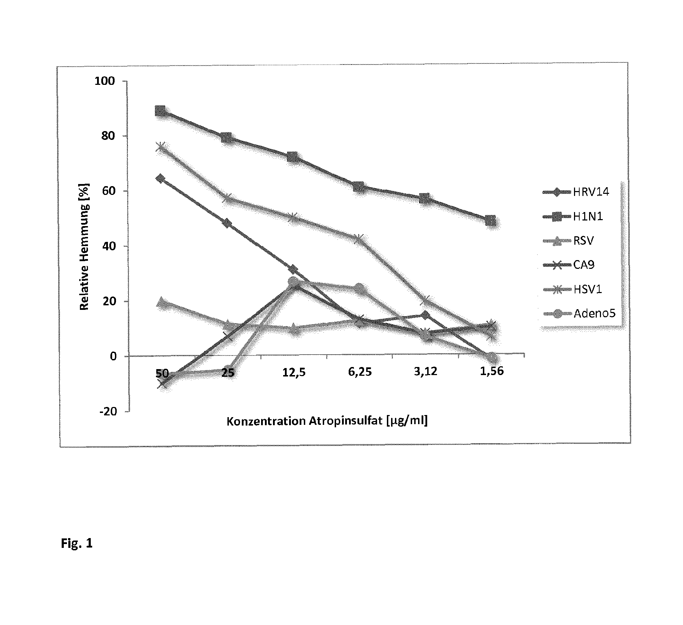

Insofern Atropin oder sein Salz alleine zur Behandlung einer viralen Infektion eingesetzt wird, soll die (bevorzugt flüssige) Zusammensetzung zwischen 1 bis 100 µg/ml bevorzugt 12,5 bis 75 µg/ml und am meisten bevorzugt 25 bis 50 µg/ml Atropinsulfat enthalten. Selbst bei sehr niedrigen Konzentrationen kann noch eine antivirale Wirkung gegen H1N1 erzielt werden (siehe Fig. 1: 1,56 µg/ml). Falls Atropinsulfat in Kombination mit ein oder mehreren der weiteren Bestandteile der pharmazeutischen Zusammensetzung verwendet wird, wird regelmäßig von einer eher niedrigeren Dosierung von Atropinsulfat auszugehen sein (siehe unten).

Erfindungsgemäß hat sich herausgestellt, dass Atropinsulfat alleine in den angegebenen Konzentrationen besonders gut zur Bekämpfung einer viralen Infektion durch Herpesviren, RSV-Viren, Rhinoviren oder Influenzaviren eingesetzt werden kann.

Als weiterer Bestandteil der pharmazeutischen Zusammensetzung kann Aconitin zum Einsatz kommen. Aconitin ist ein Diterpenoid-Alkanoid, das die Inaktivierung des spannungsabhängigen Natriumkanals verlangsamt und dadurch den Einstrom von Natriumionen während des Aktionspotentials der Nerven verlängert. Es wirkt insofern zunächst zentral wie peripher auf motorische wie sensible Nerven zunächst erregend gefolgt von einer Lähmung. Kardiale Auswirkungen können Arrhythmien, Bradykardie sowie diastolischen Herzstillstand mit sich bringen. Die dosis letalis von Aconitin liegt bei Erwachsenen bei ca. 5 mg. Aconitin weist folgende Strukturformel auf:

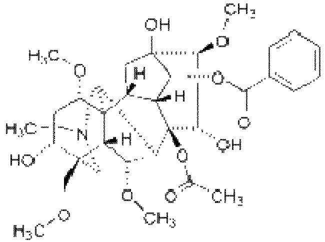

Die chemische Summenformel lautet C₃₄H₄₇NO₁₁. Eine antivirale Wirksamkeit von Aconitin wurde bislang nicht beschrieben.

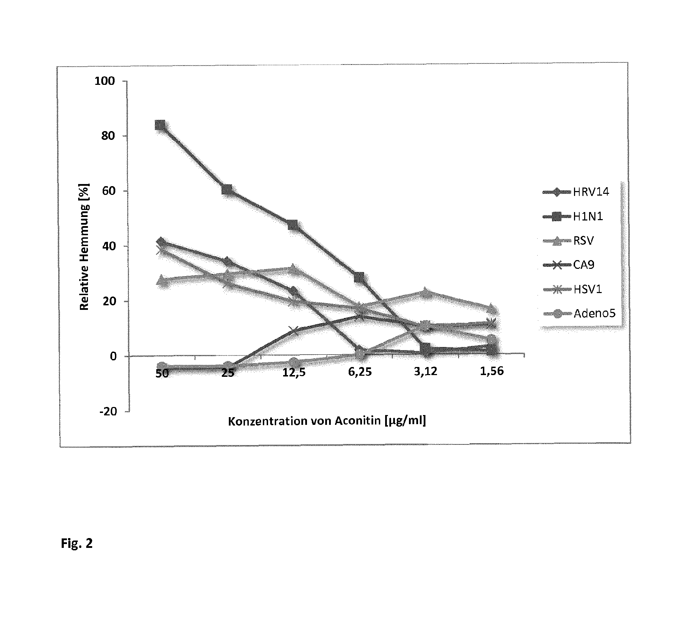

Falls die pharmazeutische Zusammensetzung Aconitin als einzigen wirksamen Bestandteil umfassen sollte wird die jeweils in Lösung eingesetzte Konzentration zur peroralen Verabreichung zwischen 0,5 - 100 µg/ml, bevorzugt 12,5 - 75 µg/ml und am meisten bevorzugt 25 - 50 µg/ml Aconitin enthalten. Eine derartige Zusammensetzung kann insbesondere zur Behandlung einer durch Herpesviren, RSV-Viren und Influenzaviren hervorgerufenen viralen Infektion eingesetzt werden. Es sei hierbei insbesondere auf den Abschnitt Beispiele sowie auf Fig. 2 verwiesen, die die antivirale Wirksamkeit verschiedener Konzentrationen von Aconitin wiedergibt.

Der dritte Bestandteil der erfindungsgemäßen pharmazeutischen Zusammensetzung ist Quecksilbercyanid (Hg(CN)₂). Quecksilbercyanid ist eine hochgiftige Verbindung, die lediglich in der Homöopathie zur Behandlung von Diphterie, schwerer Angina und Erkältungen verwendet wird. Eine spezielle antivirale Wirksamkeit ist jedoch bislang nicht bekannt.

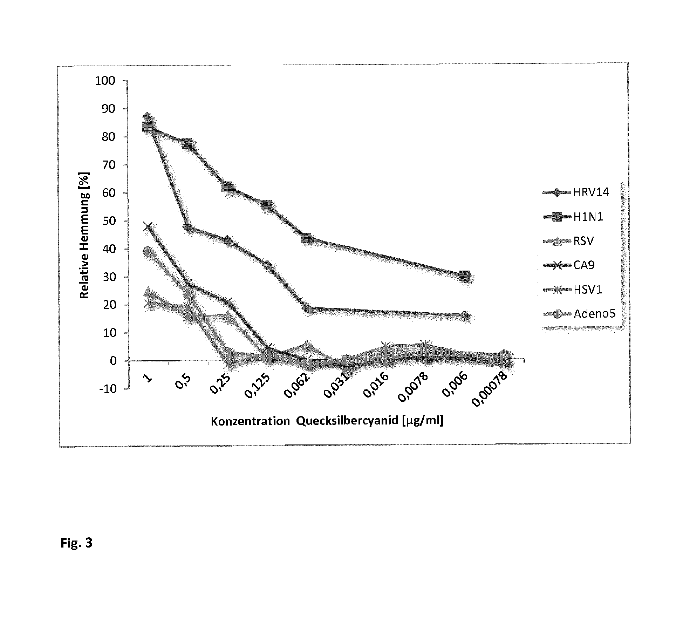

Quecksilbercyanid wird, falls es als einzige Komponente der erfindungsgemäßen Zusammensetzung verwendet wird, in Konzentrationen zwischen 0,001 bis 10 µg/ml, bevorzugt 0,1 bis 5 µg/ml, am meisten bevorzugt 0,5 - µg/ml Quecksilbercyanid in Lösung eingesetzt. Erfindungsgemäß hat sich herausgestellt, dass diese Substanz insbesondere zur Behandlung von durch Adenoviren, Rhinoviren, Enteroviren oder Influenzaviren hervorgerufenen viralen Infektionen eingesetzt werden kann. Detaillierte Ergebnisse bezüglich der antiviralen Wirksamkeit von Quecksilbercyanid in verschieden Konzentrationen sind in Fig. 3 dargestellt.

Gemäß einem weiteren Aspekt der vorliegenden Erfindung umfasst die pharmazeutische Zusammensetzung eine Kombination aus Atropin (oder einem Salz hiervon), insbesondere Atropinsulfat, sowie Aconitin und Quecksilbercyanid. Für eine derartige Zusammensetzung hat es sich als vorteilhaft herausgestellt, die Konzentration der jeweiligen Einzelbestandteile niedriger anzusetzen als bei einer Monotherapie. So können die erfindungsgemäße pharmazeutische Zusammensetzung, beispielsweise in Form einer wässrig/ethanolischen Lösung ungefähr 1-10, vorzugsweise 5 µg/ml Atropinsulfat, ungefähr 0,5 - 5, vorzugsweise 1, µg/ml Aconitin und ungefähr 0,001 - 0,1 µg/ml, vorzugsweise 0,004 µg/ml, Quecksilbercyanid enthalten.

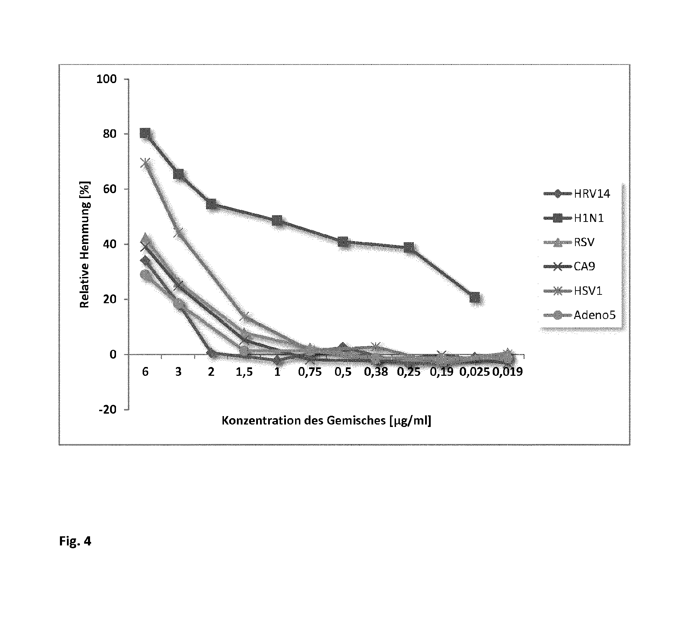

Eine besonders bevorzugte Zusammensetzung umfasst eine Lösung aus Ethanol, Glycerol und Wasser, die ungefähr 5 µg/ml Atropinsulfat, µg/ml Aconitin sowie 0,004 µg/ml Quecksilbercyanid enthält. Eine derartige Zusammensetzung ist bereits, wie in der Einleitung geschildert, bekannt und wird unter der Bezeichnung "Meditonsin^{®}" seit Jahrzehnten erfolgreich auf dem europäischen Markt vertrieben. Erstaunlicherweise hat sich herausgestellt, dass die Verwendung der Dreierkombination gegenüber der antiviralen Aktivität der Einzelkomponenten eine synergistische Wirkung entfaltet. Wie sich Fig. 4 entnehmen lässt, ist die so genannte "Meditonsin"-Mischung insbesondere gegen RSV, CA9, HSV1 und H1N1, jedoch auch gegen Adeno5 sowie HRV14 stark bis sehr stark antiviral wirksam. Dies insbesondere unter Berücksichtigung der Tatsache, dass die in der Mischung eingesetzten Konzentrationen der Einzelkomponenten niedriger sind als die jeweils für die Einzelversuche eingesetzten Mengen.

Die Gesamtzusammensetzung umfassend oder bestehend aus allen drei Bestandteilen kann insbesondere zur Bekämpfung einer durch Influenzaviren hervorgerufenen viralen Infektion eingesetzt werden. Jedoch ist auch ein Einsatz gegen Herpes simplex Viren und andere Viren denkbar.

Die pharmazeutische Zusammensetzung der vorliegenden Erfindung (Mischung) wird in einer täglichen Gesamtdosis von ungefähr 20 µg/ml Atropinsulfat, 4 µg/ml Aconitin und 0,016 µg/ml Quecksilbercyanid an den erwachsenen Patienten verabreicht. Abweichungen von ca. ± 30 % von der angegebenen Menge sind hierbei tolerabel.

Bei Einsatz der Einzelkomponenten Atropin, Aconitin und Quecksilbercyanid jeweils alleine erhöhen sich die einzusetzenden Dosen im Verhältnis zu den oben für die Einzelsubstanzen angegebenen Konzentrationen.

Mit anderen Worten werden einem erwachsenen Patienten ca. 2 - 6 ml der pharmazeutischen Zusammensetzung pro Tag verabreicht, wobei die Zusammensetzung die oben angegebenen Konzentrationen aufweist. Die Dosierung halbiert sich für Kinder zwischen dem 6. und 12. Lebensjahr, wird für Kleinkinder ab dem 1. Lebensjahr noch weiter reduziert und beträgt ungefähr 25 % für Säuglinge ab dem 7. Lebensmonat.

Die vorliegende Erfindung wird nunmehr unter Bezugnahme auf die nachfolgenden Ausführungsbeispiele erläutert, in denen die Effekte der erfindungsgemäßen Zusammensetzung dargelegt werden.

### Beispiele:

### Material und Methoden

### Prüfsubstanzen

Prüfsubstanzen waren Atropinsulfat (AT), Aconitin (AC) und Quecksilbercyanid (QC) sowie eine Mischung aus AT, AC und QC. AT und AC lagen in fester Form vor, QC als fertige Gebrauchslösung (10 mg/ml, 43 % Ethanol).

### Vorbereitung der Prüfmuster

Aus AT, AC und QC wurden mithilfe von Ethanol und Komplettmedium die Prüflösungen in einer Konzentration von jeweils 1mg/ml hergestellt. Außerdem wurden alle drei Prüfsubstanzen (AT und AC: jeweils 1mg/ml; QC: 0,001 µg/ml) im Verhältnis 5:1:4 (AT:AC:QC) gemischt und auf eine Konzentration von 1mg/ml (Gesamtsubstanz) verdünnt. Aus jeder Prüflösung wurden Verdünnungsreihen (log2-Stufen) hergestellt und diese auf Zytotoxizität getestet. Die höchste, nicht mehr zytotoxische Verdünnungsstufe wurde als Ausgangskonzentration für weitere Verdünnungsreihen (ebenfalls log2-Stufen) genutzt, die im weiteren Verlauf der Untersuchung auf antivirale Wirkung getestet wurden.

### Referenzsubstanzen

Als Referenzsubstanzen zur Verifikation des Testsystems wurden folgende handelsübliche antivirale Wirkstoffe eingesetzt: Ribavirin (Viracole^{®}) bei RSV, Aciclovir (Zovirax^{®}) bei HSV1, Amantadinhydrochlorid (AMA) bei H1N1 und Oxymetazolinhydrochlorid (OMZ) bei HRV14. Die Effektivität der Referenzsubstanzen konnte im Testsystem bestätigt werden. Eine Konzentration von 5 µg Ribavirin pro ml bzw. 2,5 µg Aciclovir pro ml bzw. 5 µg AMA pro ml bzw. 10 µg OMZ pro ml bei eingesetzter M. O.I. (mittlere Infektionsdosis) von 0,0004 erbrachten durchschnittlich eine Reduktion der Infektiosität von etwa 50-60 %. Im Falle von CA9 und Adeno5 wurden laborinterne Standards mitgeführt, die ebenso im Testsystem bestätigt werden konnten. Die laborinternen Standards wurden dabei so eingestellt, dass eine 50- bis 70%ige Reduktion der Infektiosität nachgewiesen werden konnte.

### Zellen und Viren

Die getesteten Viren umfassten behüllte und unbehüllte RNA- und DNA-Viren. Folgende Viren wurden verwendet: HRV14 (humanes Rhinovirus Typ 14; unbehülltes RNA-Virus), RSV (humanes Respiratory-Syncytial-Virus, Stamm Long; behülltes RNA-Virus), HSV1 (Herpes-simplex-Virus Typ 1; behülltes DNA-Virus), Adeno5 (humanes Adenovirus 5; unbehülltes DNA-Virus), CA9 (humanes Coxsackie-Virus Typ 9; unbehülltes RNA-Virus) und H1N1 (humanes Influenza-A-Virus; behülltes RNA-Virus). HRV14 wurden in HeLa-Zellen eingeführt, RSV, HSV1 und Adeno5 in HEp-2-Zellen, CA9 in BGM-Zellen und H1N1 in MDCK-Zellen.

### Zytotoxizitätstests

Die In-vitro-Zytotoxizität der Prüfsubstanzen wurde an physiologisch aktiven Zellen analysiert mithilfe des MTT-Tests, welcher die Quantifizierung der Aktivität mitochondrialer Enzyme in aktiven und sich teilenden Zellen in direkter Korrelation zwischen Lebensfähigkeit und Enzymaktivität ermöglicht. Um die höchstmögliche Konzentration an Prüfsubstanz zu bestimmen, die nicht mehr zytotoxisch wirkt, wurden nicht-vireninfizierte HeLa-, BGM-, HEp-2- und MDCK-Zellen zusammen mit den Prüflösungen in verschiedenen Verdünnungen (log2-Verdünnungen von 1000 µg/ml hinunter bis zu 1,95 µg/ml) bei 37°C und 5%iger CO₂-Begasung für mindestens 5 Tage kultiviert. Entsprechende Zellkulturmedien ohne Prüfsubstanz wurden als Kontrolle mitgeführt (Mediumkontrolle).

### Tests der antiviralen Aktivität

### Durchführung

Die antivirale Aktivität der Prüfsubstanzen wurde mittels der Analyse des zytopathogenen Effekts (CPE) im Falle derAdeno5-Viren bzw. mittels des Plaquereduktionstests als PFU (plaque-forming units) bei allen anderen Viren gemessen. Die Zellmonolayer wurden mit einer mittleren Infektionsdosis (M.O.I.) von 0,0004 (RSV, HSV1, HRV14, H1N1) bzw. 0,0003 (CA9) infiziert. Bei Adeno5 sowie zusätzlich bei HRV14 und H1N1 wurde eine höhere Infektionsdosis von 0,008 bzw. 0,0006 verwendet. Die Zellen wurden für jeweils 1h bei 34°C infiziert, danach gewaschen und mit Zellkulturmedium überschichtet, das die jeweilige Testsubstanz in verschiedenen, nicht zelltoxischen Konzentrationen (log2-Verdünnungsreihe) enthielt. Die Testansätze wurden so lange kultiviert, bis in den unbehandelten Viruskontrollen mikroskopisch die eingestellte Plaquezahl zu beobachten war.

### Berechnung der antiviralen Aktivität

Die antivirale Aktivität wurde anhand der Plaquezahl, der Läsionen von viralem CPE (Adeno5) und der Menge an viralem Protein (Adeno5 und RSV) quantifiziert. Dabei wurden die infizierte "helle" Zellfläche (bei CPE) bzw. die Plaquezahl (bei PFU) bzw. der im ELISA ermittelten Extinktionswerte (Immuntest, OD = 492 nm) der jeweiligen unbehandelten Kontrolle als 100%ige Infektion definiert. Demgegenüber wurden die Zellflächen, die Plaquezahlen sowie die OD der jeweiligen Testansätze ausgewertet, sodass hemmende Effekte der zu analysierenden Substanzen als prozentuale Substanzhemmung gezeigt werden konnten.

Die Berechnung von EC₅₀ (mittlere effektive Konzentration) und anderen wichtigen Werten erfolgte durch nichtlineare Regression. Im Falle einer maximalen Virushemmung von weniger als 50% wurden EC₅₀ -Werte als >100 µg/ml bzw. >120 µg/ml ausgegeben.

### Klassifizierung der antiviralen Aktivität

Die antivirale Aktivität der Prüfsubstanzen bzw. der Mischung wurde in die Kategorien schwach, mäßig, stark und sehr stark eingeteilt. Die Kategorisierung wurde entsprechend der prozentualen Hemmung vorgenommen. "Schwach" entsprach 0-25% Hemmung, "mäßig" 25-50% Hemmung, "stark" 50-75% und "sehr stark" 75-100%.

### Ergebnisse

### Zytotoxizität von Atropinsulfat, Aconitin und Quecksilbercyanid

Die mittlere inhibitorische Konzentration (IC₅₀) betrug je nach Zeillinie im Mittel zwischen 234,4 und 339,5 µg/ml für AT und zwischen 262,5 und 725,0 µg/ml für AC. Eine sehr viel niedrigere IC50 zeigte Quecksilbercyanid (QC) mit Mittelwerten zwischen 8,3 und 20,8 µg/ml. Die 5:1:4-Mischung hatte einen IC₅₀-Wert von 15,6 µg/ml bei BGM-Zellen bzw. von 20,8 µg/ml bei HEp-2-Zellen (s. Tabelle 1).

Für die antivirale Prüfung wurden die Prüflösungen auf einen Wert von 50 µg Prüfsubstanz pro ml bei AT und AC und auf µg/ml bei QC als Ausgangskonzentration für die weiteren log2-Verdünnungsreihen eingestellt. Die entsprechend verwendete Ausgangskonzentration der laborintern hergestellten "Meditonsin"-Mischung für die antivirale Prüfung betrug 6,004 µg Gesamtsubstanz pro ml.

### Antivirale Aktivität der "Meditonsin"-Mischung

Die laborintern hergestellte Mischung aus Atropinsulfat, Aconitin und Quecksilbercyanid ("Meditonsin"-Mischung) bestand aus 5 µg/ml Atropin, µg/ml Aconitin und 0,004 µg/ml Quecksilbercyanid. Diese Mischung wirkte mäßig bis sehr stark hemmend auf alle Viren (RSV: 42,4 %; CA9: 39,2 %; HSV1: 69,5 %; Adeno5: 29,0 %; HRV14: 34,0 %; H1N1: 80,3 %). Die stärkste Hemmung war auf die H1N1-Viren zu beobachten (s. Fig. 4). Mit zunehmender Verdünnung verringert sich die antivirale Wirkung merklich.

Interessant ist der Vergleich mit den synthetischen Virostatika, die bei den Tests als Kontrolle stets mit durchgeführt worden sind (s. Tabelle 3). Die "Meditonsin"-Mischung hatte ab 6 µg/ml einen mindestens so starken inhibitorischen Effekt gegen H1N1 in niedriger Infektionsdosis (M.O.I. = 0,0004) wie Amantadin (5 µg/ml). Des Weiteren wirkte die "Meditonsin"-Mischung (6 µg/ml) stärker antiviral gegen den Herpesvirus HSV1 als Aciclovir (2,5 µg/ml).

### Antivirale Aktivität von Atropinsulfat, Aconitin und Quecksilbercyanid

Die Einzelkomponenten der "Meditonsin"-Mischung zeigten ebenfalls einen antiviralen Effekt, dessen Stärke in Abhängigkeit vom Virus und vom Verdünnungsgrad variierte. In hohen, nicht zytotoxischen Konzentrationen entfaltete Atropin eine starke bis sehr starke antivirale Wirkung gegen H1N1, HSV1 und HRV14 (Fig. 1), während Aconitin vor allem gegen H1N1 und Quecksilbercyanid vor allem gegen HRV14 und H1N1 antiviral wirkten (s. Tabelle 2 und Fig. 2).

Die Einzelkomponenten haben an der antiviralen Gesamtwirkung der "Meditonsin"-Mischung je nach Virus einen unterschiedlichen Anteil. So dominiert bei der antiviralen Wirkung der Mischung gegen H1N1, Adeno5 und HSV1 das Atropin eindeutig, während gegen RSV und CA9 Atropin und Aconitin gemeinsam das meiste zur dieser Wirkung beitragen. Gegen HRV14 wirken Atropin und Quecksilbercyanid in der Mischung dominierend. Darüber hinaus spielt Quecksilbercyanid auch bei der antiviralen Wirkung der Mischung gegen H1N1 eine starke Rolle (s. Fig. 3).

Auch im Vergleich mit den synthetischen Virostatika, die als Kontrolle bei den In-vitro-Tests mitgeführt wurden, zeigten die Einzelkomponenten in Abhängigkeit von deren Konzentration eine vergleichbare antivirale Wirkung (s. Tabelle 3). So wirkte Atropin ab 25 µg/ml mindestens so stark antiviral gegen HSV1 wie Aciclovir (2,5 µg/ml), ab 50 µg/ml mindestens so stark antiviral gegen HRV14 in niedriger Infektionsdosis (M.O.I. = 0,0004) wie Oxymetazolinhydrochlorid (10 µg/ml) und ab 12,5 µg/ml mindestens so stark antiviral gegen H1N1 in niedriger Infektionsdosis (M.O.I. = 0,0004) bzw. ab 25 µg/ml mindestens so stark antiviral gegen H1N1 in höherer Infektionsdosis (M.O.I. = 0,0006) wie Amantadin (5 µg/ml).

Ähnliches gilt auch für Aconitin und Quecksilbercyanid. Aconitin ab 50 µg/ml wirkte mindestens so stark antiviral gegen H1N1 in niedriger Infektionsdosis bzw. ab 50 µg/ml mindestens so stark antiviral gegen H1N1 in höherer Infektionsdosis wie Amantadin (5 µg/ml). Quecksilbercyanid ab 1 µg/ml wirkte mindestens so stark antiviral gegen HRV14 in niedriger und höherer Infektionsdosis wie Oxymetazolinhydrochlorid (10 µg/ml) und ab 0,5 µg/ml mindestens so stark antiviral gegen H1N1 in niedriger Infektionsdosis bzw. ab einer Konzentration von µg/ml mindestens so stark antiviral gegen H1N1 in höherer Infektionsdosis wie Amantadin (5 µg/ml).

**Tabelle 1: Zytotoxizität von Atropinsulfat (AT), Aconitin (AC), Quecksilbercyanid (QC) und einer 5:1:4-Mischung aus AT, AC und QC, angegeben als IC₅₀ in der Einheit [µg/ml]. IC = inhibitory concentration.**

| | **MDCK** | **HEp-2** | **HeLa** | **BGM** |
|---|---|---|---|---|
| AT | 234,4 | 239,5 | 333,3 | 309,0 |
| AC | 300,0 | 375,0 | 262,5 | 725,0 |
| QC | 19,5 | 14,6 | 8,4 | 8,28 |
| Mischung | - | 20,8 | - | 15,6 |

**Tabelle 2: Prozentuale Hemmung der Virenaktivität unter Einfluss von hochkonzentrierten, nicht zytotoxischen Prüflösungen aus Atropinsulfat (AT, 50 µg/ml), Aconitin (AC, 50 µg/ml) und Quecksilbercyanid (QC, µg/ml). M.O.I. = multiplicity of infection, PFU = plaque forming units.**

| **Virus** | **Zellkultur** | **M.O.I.** | **Assay** | **Prozentuale Hemmung** | | |
|---|---|---|---|---|---|---|
| | | | | **AT** | **AC** | **QC** |
| RSV | HEp-2 | 0,0004 | PFU | 19,88 | 27,67 | 24,73 |
| HSV1 | HEp-2 | 0,0004 | PFU | 75,95 | 38,54 | 20,55 |
| Adeno5 | HEp-2 | 0,008 | CPE | -6,67 | -3,90 | 38,89 |
| CA9 | BGM | 0,0003 | PFU | -10,04 | -4,58 | 47,88 |
| HRV14 | HeLa | 0,0004 | PFU | 64,41 | 41,48 | 86,90 |
| H1N1 | MDCK | 0,0004 | PFU | 89,01 | 83,87 | 83,33 |

In diesem Zusammenhang wird auch auf Fig. 4 verwiesen: Gezeigt wird die prozentuale Hemmung der viralen Aktivität der "Meditonsin"-Mischung. Die Konzentration der Ausgangsmischung beträgt 6,004 µg/ml Gesamtsubstanz. Gezeigt ist die antivirale Wirksamkeit der Ausgangsmischung sowie von Mischungen mit abnehmender Gesamtkonzentration. Erkennbar zeigt auch eine Mischung mit einer Gesamtkonzentration von 2-3 µg/ml gegen einige Viren eine bemerkenswerte Hemmwirkung.

**Tabelle 3: Mindestkonzentrationen in µg/ml von Atropin, Aconitin, Quecksilbercyanid und der "Meditonsin"-Mischung, die notwendig sind, um eine vergleichbare antivirale Wirkung zu erreichen wie die entsprechenden antiviralen Chemotherapeutika (Standard).**

| **Virus** | **Standard*)** | **Atropin** | **Aconitin** | **Quecksilbercyanid** | **Mischung** |
|---|---|---|---|---|---|
| HSV1 | 2,5 | 25 | - | - | 6 |
| HRV14 | 10 | 50 | - | 1 | - |
| H1N1 | 5 | 12,5 | 50 | 0,5 | 6 |

| | | | | | |
|---|---|---|---|---|---|
| *) Standards: Aciclovir bei HSV1, Oxymetazolinhydrochlorid bei HRV14, Amantadin bei H1N1 | | | | | |

### Diskussion

Die vorliegende Erfindung zeigt die antiviralen Eigenschaften der Wirkstoffe Atropin, Aconitin und Quecksilbercyanid sowie einer daraus laborintern hergestellten Mischung, die in der Zusammensetzung Meditonsin^{®} nachempfunden war, zum ersten Mal. Es konnte nachgewiesen werden, dass sowohl die Mischung als auch deren Einzelkomponenten die Aktivität aller getesteten Erkältungs-, Influenza- und Herpesviren schwach bis sehr stark hemmen. Eine ähnliche Beobachtung bezüglich des virostatischen Effekts von pflanzenbasierten Wirkstoffen und Pflanzenextrakten konnte auch in anderen Studien gezeigt werden.

Die "Meditonsin"-Mischung entfaltete die höchste antivirale Wirkung gegen den Influenza-A-Virus H1N1 und gegen den Herpes-simplex-Virus HSV1. Aber auch auf die anderen Viren, die in der vorliegenden Untersuchung verwendet wurden, war ein mäßiger virostatischer Effekt zu beobachten. In ähnlicher Weise konnte auch in anderen Studien ein spezifisches antivirales Wirkspektrum für in Komplexhomöopathika verwendete Einzelmittel beobachtet werden. Allerdings wirkte jenes virostatisch in erster Linie gegen RSV und weniger gegen Influenza-A-Viren.

In der vorliegenden Untersuchung wurde auch der interessanten Frage nachgegangen, welche Komponenten hauptverantwortlich für die antivirale Wirkung der "Meditonsin"-Mischung sind. Die Einzelkomponenten Atropin, Aconitin und Quecksilbercyanid zeigten im Test eine antivirale Wirkung und ein jeweils spezifisches antivirales Wirkspektrum. So entfalteten Atropin und Aconitin in erster Linie ihre antivirale Wirkung gegen HSV1, H1N1 und RSV, während QC gegen CA9, HRV14 und Adeno5 wirkte. Es konnte somit gezeigt werden, dass bei der antiviralen Wirkung der Mischung offenbar additive bzw. synergistische Effekte zum Tragen kommen.

## Patentansprüche

1. Pharmazeutische Zusammensetzung umfassend
a) Atropin oder ein Salz hiervon;
b) Aconitin; und/oder
c) Quecksilbercyanid;
zur Verwendung in einem Verfahren zur Behandlung einer viralen Infektion.

2. Pharmazeutische Zusammensetzung zur Verwendung in einem Verfahren zur Behandlung einer viralen Infektion nach Anspruch 1, wobei die virale Infektion durch Herpesviren, Influenzaviren, Adenoviren, Rhinoviren und/oder Enteroviren hervorgerufen wird.

3. Pharmazeutische Zusammensetzung zur Verwendung in einem Verfahren zur Behandlung einer viralen Infektion nach Anspruch 1 oder 2, wobei die virale Infektion durch HRV14 (humanes Rhinovirus Typ 14), RSV (humanes Respiratory-Syncytial-Virus), HSV1 (Herpes-simplex-Virus Typ 1), Adeno5 (humanes Adenovirus 5), CA9 (humanes Coxsackie-Virus Typ 9) und/oder H1N1 (humanes Influenza-A-Virus) hervorgerufen wird.

4. Pharmazeutische Zusammensetzung zur Verwendung in einem Verfahren zur Behandlung einer viralen Infektion nach einem oder mehreren der vorhergehenden Ansprüche, wobei die Zusammensetzung als wässrig/ethanolische Lösung vorliegt und ungefähr 5 µg/ml Atropinsulfat, ungefähr µg/ml Aconitin und ungefähr 0,004 µg/ml Quecksilbercyanid enthält.

5. Pharmazeutische Zusammensetzung zur Verwendung in einem Verfahren zur Behandlung einer viralen Infektion nach Anspruch 4, wobei die virale Infektion durch Influenza-Viren hervorgerufen wird.

6. Pharmazeutische Zusammensetzung zur Verwendung in einem Verfahren zur Behandlung einer viralen Infektion nach einem oder mehreren der Ansprüche 1-3, wobei die Zusammensetzung zwischen 1-100 µg/ml, bevorzugt 12,5-75 µg/ml, am meisten bevorzugt 25-50 µg/ml Atropinsulfat enthält.

7. Pharmazeutische Zusammensetzung zur Verwendung in einem Verfahren zur Behandlung einer viralen Infektion nach Anspruch 6, wobei die virale Infektion durch Herpesviren, RSV-Viren, Rhinoviren oder Influenzaviren hervorgerufen wird.

8. Pharmazeutische Zusammensetzung zur Verwendung in einem Verfahren zur Behandlung einer viralen Infektion nach einem oder mehreren der Ansprüche 1-3, wobei die Zusammensetzung zwischen 0,5-100 µg/ml, bevorzugt 12,5-75 µg/ml, am meisten bevorzugt 25-50 µg/ml Aconitin enthält.

9. Pharmazeutische Zusammensetzung zur Verwendung in einem Verfahren zur Behandlung einer viralen Infektion nach Anspruch 8, wobei die virale Infektion durch Herpesviren, RSV-Viren oder Influenza-Viren hervorgerufen wird.

10. Pharmazeutische Zusammensetzung zur Verwendung in einem Verfahren zur Behandlung einer viralen Infektion nach einem oder mehreren der Ansprüche 1-3, wobei die Zusammensetzung zwischen 0,001 und 10 µg/ml, bevorzugt 0,1-5 µg/ml, am meisten bevorzugt 0,5-1 µg/ml Quecksilbercyanid enthält.

11. Pharmazeutische Zusammensetzung zur Verwendung in einem Verfahren zur Behandlung einer viralen Infektion nach Anspruch 10, wobei die virale Infektion durch Adenoviren, Rhinoviren, Enteroviren oder Influenzaviren hervorgerufen wird.

12. Pharmazeutische Zusammensetzung zur Verwendung in einem Verfahren zur Behandlung einer viralen Infektion nach einem oder mehreren der vorhergehenden Ansprüche, wobei die Zusammensetzung in einer täglichen Dosis von ungefähr 20 µg/ml Atropinsulfat, 4 µg/ml Aconitin und 0,016 µg/ml Quecksilbercyanid verabreicht wird.
